# EUROPEAN PATENT APPLICATION

(11) **EP 3 827 816 A1**
(43) Date of publication of application: **02.06.2021**
(21) Application number: 19212199.4
(22) Date of filing: 28.11.2019
(51) Int. Cl.: A61K 31/095, A61K 31/10, A61K 31/275, A61P 33/00, A61P 33/02, A61P 35/00

(54) **PROOXIDATIVE CHAIN-TRANSFER AGENTS FOR USE IN THE TREATMENT OF MALIGNANT TUMOUR OR INFECTIOUS DISEASES**

(71) Applicant: Universitätsmedizin der Johannes Gutenberg-Universität Mainz, 55131 Mainz (DE)
(72) Inventor: MOOSMANN, Bernd, 55128 Mainz (DE)
(74) Representative: Patentanwälte Dr. Keller, Schwertfeger Partnerschaft mbB

(57) **Abstract**

The present invention relates to prooxidative chain-transfer agents for use in the treatment of a malignant tumour disease, or infectious disease. The prooxidative chain-transfer agents are selected from lipophilic thiols, lipophilic trithiocarbonates, lipophilic, aromatic dithioesters, and lipophilic, aromatic thiols. The compounds amplify the prooxidative activity at the target site and are therefore highly efficient and specific for their targets.

## Description

The present invention relates to prooxidative chain-transfer agents (CTAs) for use in the treatment of a malignant tumour disease, or infectious disease.

The currently known treatment strategies for tumour diseases or infectious diseases that are based on a more detailed understanding of the underlying biology are expected to improve the treatment outcome for patients suffering from these diseases. However, many of these strategies still have one common and critical problem, being their limited specificity for tumour cells or parasitic cells.

The three major types of cancer treatment, i.e. surgery, chemotherapy and radiotherapy, are powerful, but are associated with risks of injury or toxicity to healthy tissues. Therefore, novel treatment methods having the ability to kill tumour cells without exhibiting these side-effects undergo extensive research and clinical studies. Among them, photodynamic therapy (PDT), sonodynamic therapy (SDT), T-cell immunotherapy and oncolytic virotherapy are expected to improve the treatment outcomes, because they are based on novel target cell killing mechanisms.

A number of medical treatments are available for the treatment of malignant tumours that yielded in a dramatic improvement in cancer survivorship around the world (Pardee and Stein, 2009). The most common active substances that are used in general pharmacotherapy of tumours are cytostatic and cytotoxic substances such as alkylating or intercalating agents, platinum compounds, antimetabolites of the nucleotide metabolism, or topoisomerase and mitose inhibitors that act on the genomic DNA and the cytoskeleton, respectively (Sessa et al., 2012; Aktories et al., 2017). In addition, low molecular weight or antibody-based antagonists of angiogenesis and hormonal stimulation and growth factor stimulation of tumour cell division are also known classes of agents that are frequently used in cancer therapy. In addition, there are a number of very specific therapies available for cases of individual degenerations, for example a degeneration of the haematological system (Sessa et al., 2012; Aktories et al., 2017).

Parasites are microorganisms that live on or inside another organism known as the host organism and benefit at the expense of their host organism. Parasites are responsible for billions of human infections, including malaria. Parasitic infections are especially prevalent in tropical areas, but they also occur in subtropical and temperate regions, where they tend to infect immigrants and travelers. While parasites can include a diverse array of microorganisms, including fungi and bacteria, medically-relevant parasites known to cause disease in humans are protozoa, helminths, and ectoparasites.

The protozoa that are infectious to humans can be classified into four groups based on their mode of movement: Sarcodina, Mastigophora, Ciliophora and Sporozoa. The three main groups of helminths that are human parasites are flatworms (platyhelminths) including trematodes (flukes) and cestodes (tapeworms), thorny-headed worms (acanthocephalans) and roundworms (nematodes). Ectoparasites comprise blood-sucking arthropods such as mosquitoes, but also other organisms such as ticks, fleas, lice, and mites that attach or burrow into the skin and remain there for a few weeks or months.

Parasitic nematodes are responsible for widespread morbidity in humans and animals. It is estimated that approximately 1.5 billion people are infected with one or more of these organisms (Hotez et al., 2008; World Health Organization, 2017) which also pose a considerable burden for animal production (Eijck and Borgsteede, 2005; Nganga et al., 2008). The extraordinary success of these parasites is to a large extent based on their ability to withstand a multitude of stresses such as host immune pressures and infectious challenges from microbes. Most parasitic nematodes inhabit the intestines of their hosts, co-existing with numerous microbial species. In studying these dynamics, research was mainly focused on the host-parasite relationship and only recently, the role of the microbiota as a major third party in said relationship is better appreciated due to diverse and far-reaching influences in health and disease (Donaldson et al., 2016). Much attention was given to host immune mechanisms while the interactions between nematodes and their microbial environments were largely overlooked. Due to many technical and biological challenges associated with studying parasites, these questions still remain difficult to address.

The roundworm *Caenorhabditis elegans* (*C. elegans*) has risen to the status of a top model organism for biological research in the last fifty years (Frézal and Félix, 2015). *C. elegans* has been very well characterized and its interactions with bacteria have been studied in considerable detail. As such, these findings might be conveyed to parasitic nematodes in general, and greatly inform our understanding of how parasites interact with the host-microbiota, as many immune-related pathways and responses may be conserved (Tarr, 2012; Rosso et al., 2013).

Antiparasitic drugs have been developed to manage infections caused by various protozoa, helminths, and ectoparasites. The individual treatment options vary, and they mainly depend on the specific causative organism within each group. However, there are several drugs that are commonly used in the treatment of different types of parasite infections in both humans and animals. For example, metronidazole has been found as an efficient drug that is active both against parasites and bacteria (Löfmark et al., 2010). Chloroquin and Artemisinin have been found to be efficient against plasmodia (Tse et al., 2019), albendazole has been found to be effective against roundworms and praziquantel against tapeworms (Albonico et al., 2015; Aktories et al., 2017). Benznidazole is a nitroimidazole antiparasitic with good activity against acute infection with *Trypanosoma cruzi,* commonly referred to as Chagas disease.

Overall, the pharmaceutical options available for the treatment of parasitic infections that are caused by either single-cellular or multi-cellular organisms is limited. Pharmaceutically active compounds are often based on a few known lead structures, which are sometimes only moderately specific in their effect (Löfmark et al., 2010; Albonico et al., 2015). In addition, the development of resistances against new drugs in the years following their introduction is often responsible for a reduction of their efficacy. Reduced efficacy has been observed in patients suffering from malaria (Greenwood, 2014; Kavishe et al., 2017), or worm infections in humans and animals (Krücken et al., 2017; Lanusse et al., 2018). The undesirable side effects of antiparasitic drugs are a major drawback in their clinical application, and hence are a frequent cause for forcing the physician to stop treatment. The most frequent adverse effects observed are anorexia, psychic alterations, loss of weight, excitability, sleepiness, digestive manifestations such as nausea or vomiting, and occasionally diarrhoea and intestinal colic. In the case of benznidazole, skin manifestations are the most notorious (e.g., hypersensitivity, dermatitis with cutaneous eruptions, generalized oedema, fever, lymphoadenopathy, articular and muscular pain), with depression of bone marrow, thrombocytopenic purpura and agranulocytosis being the more severe manifestations.

Many parasites are known for a weakly expressed anti-oxidative defense that goes along with similar or higher prooxidative activity as compared to normal human cells (Mehlotra, 1996; Turrens, 2004). Accordingly, if subjected to prooxidative amplification, parasitic cells are supposed to be more severely damaged than the body's own cells.

The known prooxidative therapies for treating parasitic infections apply substances such as peroxides (artemisinin) or nitroimidazoles (metronidazole). However, the specificity of those drugs depends on the binding of specific proteins or the activation of specific reductases (Li et al., 2005; Löfmark et al., 2010). Similar drawbacks have also been reported for chloroquine (Kavishe et al., 2017). There is no known drug which is able to exhibit a local prooxidative activity by reversible activation. Many drugs that have been developed to treat neurodegenerative diseases failed to gain approval for clinical use because they are not well tolerated in humans. As a result, there are strategies that are based on the principle that drugs are activated by the pathological state that they are intended to inhibit (Lipton, 2007). Examples of this approach are the potentially neuroprotective drug and glutamate receptor antagonist memantine.

Similar problems also occur in tumour therapy that faces side effects of their anti-cancer drugs that are manifesting as toxic reactions due to alkylation of proteins, or their lack of selectivity if it comes to differentiate between tumour cells and healthy cells (Sessa et al., 2012; Aktories et al., 2017). The specificity of most tumour drugs is still only based on tumour cell division or tumour antigen presentation (Pardee and Stein, 2009; Trachootham et al., 2009). Antibody-therapies that exhibit no cytostatic or only little side effects have been established for only a few tumour types, and they are extremely cost-intense (Sessa et al., 2012; Dolgin, 2018). With the development of nanotechnology, nano-drug systems offer longer blood circulation times and lower systemic toxicity of anticancer drugs (Collins , 2006).

In spite of the availability of complex therapeutic approaches, it is still not possible to ensure a satisfactory five-year survival rate for many tumour types (Siegel et al., 2019). Therefore, there is a need in tumour therapy for low-molecular weight pharmaceuticals that have a fundamentally new mechanism of action.

Tumour cells have also been known for a long time for their high prooxidative activity if compared with normal body cells (Szatrowski and Nathan, 1991). Said activity usually remains below the deadly result for tumour cells (Trachootham et al., 2009; Gorrini et al., 2013; Sosa et al., 2013). A prooxidative therapy of tumours has essentially been proven to be successful in certain cases such as classical radiotherapy (Moss, 2007; Barker et al., 2015), photodynamic therapy (Dolmans et al., 2003) or upon application of low-molecular weight pharmaceuticals (Trachootham et al., 2009; Gorrini et al., 2013; Galadari et al., 2017). More recent work also addressed the sensitization of tumour cells to prooxidants (Toler et al., 2006; Cui et al., 2017; Kubli et al., 2019).

There are also pharmacochemical approaches. For example EP1478357 B1 describes tricyclic pyrazole derivatives, process for their preparation and their use as antitumour agents. EP1124810 B9 describes 2-amino-thiazole derivatives for treating cell proliferative disorders associated with an altered cell cycle dependent kinase activity.

There are also approaches to treat both tumour diseases and parasitic infections using one class of compounds. For example, US7247715 B2 describes ricin-like toxin variants for treatment of cancer, viral or parasitic infections.

Also chain-transfer agents were used in the synthesis of active polymeric compounds. CN106995516 A describes PHPMA and PEG polymers that have a long body circulation time, and are enriched at a tumour. The polymer can carry a drug through a covalent bond and/or a non-covalent bond, and the obtained product can further be coupled to a targeting molecule and/or a labelled molecule for preparing a drug or a detection reagent. KR101389695 B1 describes a method of killing or inhibiting growth of a microorganism in a mammal other than a human infected with the microorganism, wherein alkoxycarbonylalkylthiol is used as a chain transfer agent. One example of such a microorganism is a parasite.

However, no specific drug-based intervention has been reported so far to efficiently exploit the redox differences between tumour cells and healthy cells.

It is therefore the object of the present invention to provide new compounds that have a high selectivity and specificity for the target cells and that are suitable for use in the treatment of a malignant tumour disease or infectious disease, in particular a parasitic disease in humans or animals.

This object is solved by a prooxidative chain-transfer agent with the features of claim 1. Preferred embodiments of the invention are claimed in the sub-claims.

The present invention is based on chain-transfer agents (CTAs) that are also called modifiers or regulators that have at least one weak chemical bond. These compounds react with the free-radical side of a growing polymer chain and interrupt chain growth. In the process of chain transfer, the radical is temporarily transferred to the regulating agent, which re-initiates growth by transferring the radical to another polymer or monomer.

Chain-transfer agents are often added to control the chain length during polymer synthesis to achieve certain mechanical and processing properties. Preferred chain transfer agents comprise halogen compounds, some aromatic hydrocarbons, and thiols (mercaptans).

The invention shows for the first time that certain compounds of polymer chemistry are highly potent, and act as highly selective cytotoxins for therapeutic applications in humans and other mammals as well as in animals. The specific effect of these substances on killing parasitic cells is based on the fact that parasites are often less fortified with antioxidant defenses than human or other mammalian cells.

The prooxidative chain-transfer agents according to the invention are of low molecular weight and can be administered as part of a single or combined pharmacotherapy for treating malignant tumours or parasitic infections in humans or animals.

The inventive compounds overcome the known problem of drug resistance, are highly efficient, and at the same time are of low toxicity for the treated humans or animals. That said, the inventive compounds can be applied in a selective prooxidative therapy involving a prooxidative chain transfer activity mediated by the inventive compounds. The inventive prooxidative chain-transfer agents can be grouped into four chemical classes: lipophilic thiols, lipophilic trithiocarbonates, lipophilic aromatic dithioesters, and lipophilic, aromatic thiols. These compounds are able to exhibit their prooxidative activity directly at the site of action, i.e. at the target cells. In the absence of endogenous prooxidative activity, the substances have no effect, because they are not initiating oxidation by themselves. As demonstrated herein, the effectiveness of the compounds was not compromised by hypoxic conditions. As further shown by the present invention, tumour cells and parasitic cells are more severely damaged than the body's own cells as a result of the prooxidative amplification of the inventive chain-transfer agents, thus resulting in a specific cell death of the targeted parasitic cells or tumour cells.

The inventive lipophilic thiols, lipophilic trithiocarbonates and lipophilic, aromatic dithioesters, and lipophilic, aromatic thiols can be described by formula (I), (II) (III), and (IV), respectively:
(1) lipophilic thiols comprising the general structure (I) 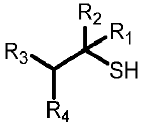 wherein R₁ - R₄ are hydrogen or aliphatic or mixed aliphatic-aromatic or mixed aliphatic-heteroatom groups in which the number of aliphatic carbon atoms is at least six: R₁ + R₂ + R₃ + R₄ ≥ C₆;
(2) lipophilic trithiocarbonates comprising the general structure (II) 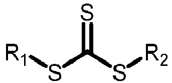
   wherein R₁ is an aliphatic or mixed aliphatic-aromatic or mixed aliphatic-heteroatom group in which the number of aliphatic carbon atoms is at least six: R₁ ≥ C₆;
   R₂ is a hydrogen or a methyl group or a substituted methyl group;
(3) lipophilic, aromatic dithioesters comprising the general structure (III) 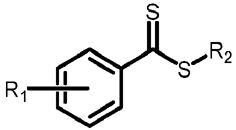
   wherein R₁ is an aliphatic or mixed aliphatic-aromatic or mixed aliphatic-heteroatom group in which the number of aliphatic carbon atoms is at least six: R₁ ≥ C₆;
   R₂ is a hydrogen or a methyl group or a substituted methyl group;
(4) lipophilic, aromatic thiols comprising the general structure (IV) 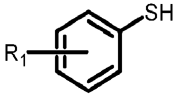 wherein R₁ is an aliphatic or mixed aliphatic-aromatic or mixed aliphatic-heteroatom group in which the number of aliphatic carbon atoms is at least six: R₁ ≥ C₆.

The inventive compounds falling under the general formulas of (I), (II), (III), (IV) are able to build thiyl radicals or sulfur radicals in the cell membrane, which makes them unique for the therapeutic and preventive applications described herein.

The term "alkyl" as used herein has 1 to 24 carbons, such as, for example, methyl, ethyl, propyl, isopropyl, butyl, t-butyl, isobutyl, pentyl, hexyl. It further comprises straight and branched chain aliphatic hydrocarbon groups such as isohexyl, heptyl, 4,4-dimethylpentyl, octyl, 2,2,4-trimethylpentyl, nonyl, decyl, undecyl, dodecyl and the like.

The term "alkoxy", as used herein has 1 to 24 carbon atoms, such as, for example methoxy or ethoxy. It also comprises straight or branched chain aliphatic hydrocarbon groups including n-propoxy, isopropoxy and the like. Preferably, the alkoxy chain is 1 to 24 carbon atoms in length, more preferably 6 to 20 carbon atoms, and more preferably 12 to 18 carbon atoms.

As used herein, the term "aryl" refers to an aromatic carbocyclic group comprising 6 to 12 carbons in the ring portion, preferably 6 to 10 carbons in the ring portion. It comprises both monocyclic or bicyclic aromatic groups such as naphthyl and tetrahydronaphthyl.

In a preferred embodiment, each R₁ - R₄ substituent comprises a hydrophobic group comprising one or more S, O or N heteroatoms in the general structure I of the lipophilic thiols. Each R₁ - R₄ substituent may be hydrophobic, but preferably includes one or more heteroatoms (S, O, N) by having a sufficient number of carbon atoms attached thereto to form a hydrophobic group. The hydrophobic group is preferably branched, substituted or unsubstituted. In a preferred embodiment, the branching occurs at the heteroatom.

Under the condition set forth above (R₁ + R₂ + R₃ + R₄ ≥ C₆), each R₁ - R₄ may be selected from the group consisting of hydrogen, hydroxyl, a substituted or unsubstituted (C1-C24) alkyl, (C1-C24) hydroxyalkyl, (C1-C24) alkyloxy-(C1-C24) alkyl, (C1-C24) alkylsulfo-(C1-C24) alkyl, (C1-C24) alkylcarboxy-(C1-C24) alkyl, (C1-C24) alkylamino-(C1-C24) alkyl, (C1-C24) alkylamino-(C1-C24) alkylamino, (C1-C24) alkylamino-(C1-C24) alkylamino-(C1-C24) alkylamino, a substituted or unsubstituted (C1-C24) aminoalkyl, a substituted or unsubstituted aryl, a substituted or unsubstituted arylamino-(C1-C24) alkyl, (C1-C24) haloalkyl, C2-C24 alkenyl, C2-C24 alkynyl, oxo, sulfo.

An aliphatic group is a branched or unbranched hydrocarbon that may be substituted or unsubstituted. Examples of branched alkyl groups include isopropyl, sec-butyl, isobutyl, tert-butyl, sec-pentyl, isopentyl, tert-pentyl, isohexyl. Substituted aliphatic groups may have one, two, three or more substituents, which may be the same or different, each replacing a hydrogen atom. Substituents are halogen (e.g., F, Cl, Br, and I), hydroxyl, protected hydroxyl, amino, protected amino, carboxy, protected carboxy, cyan, methylsulfonylamino, alkoxy, acyloxy, nitro, and lower haloalkyl.

The term "substituted" in regard of R₂ used in formula (I) refers to a methyl group having one, two, or three substituents, which may be the same or different, each replacing a hydrogen atom. Examples of substituents include but are not limited to halogen (e.g., F, Cl, Br, or I), hydroxyl, protected hydroxyl, amino, protected amino, carboxy, protected carboxy, cyan, methylsulfonylamino, alkoxy, alkyl, aryl, arylalkyl, acyloxy, and lower haloalkyl.

In an alternative embodiment relating to the general structure (II) of the lipophilic trithiocarbonates R₁ is selected from the group consisting of a substituted or unsubstituted (C6-C24) alkyl, (C6-C24) hydroxyalkyl, (C6-C24) alkyloxy, (C6-C24) alkylsulfo, (C6-C24) alkyloxy-(C6-C24) alkyl, (C6-C24) alkylsulfo-(C6-C24) alkyl, (C6-C24) alkylcarboxy-(C6-C24) alkyl, (C6-C24) alkylamino-(C6-C24) alkyl, (C6-C24) alkylamino-(C6-C24) alkylamino, (C6-C24) alkylamino-(C6-C24) alkylamino-(C6-C24) alkylamino, a substituted or unsubstituted (C6-C24) aminoalkyl, a substituted or unsubstituted aryl, a substituted or unsubstituted arylamino-(C6-C24) alkyl, (C6-C24) haloalkyl, (C6-C24) alkenyl, (C6-C24) alkynyl, and R₂ refers to a hydrogen or a methyl group having one, two, or three substituents, which may be the same or different, each replacing a hydrogen atom.

The term "substituted" in regard of R₂ used in formula (II) refers to a methyl group having one, two, or three substituents, which may be the same or different, each replacing a hydrogen atom. Examples of substituents include but are not limited to halogen (e.g., F, Cl, Br, and I), hydroxyl, protected hydroxyl, amino, protected amino, carboxy, protected carboxy, cyan, methylsulfonylamino, alkoxy, alkyl, aryl, arylalkyl, acyloxy, and lower haloalkyl.

An aliphatic group is a branched or unbranched hydrocarbon that may be substituted or unsubstituted. Examples of branched alkyl groups include isopropyl, sec-butyl, isobutyl, tert-butyl, sec-pentyl, isopentyl, tert-pentyl, isohexyl. Substituted aliphatic groups may have one, two, three or more substituents, which may be the same or different, each replacing a hydrogen atom. Preferred substituents are halogen (e.g., F, Cl, Br, or I), hydroxyl, protected hydroxyl, amino, protected amino, carboxy, protected carboxy, cyan, methylsulfonylamino, alkoxy, acyloxy, nitro, and lower haloalkyl.

In an alternative embodiment relating to the general structure (III) of the lipophilic, aromatic dithioesters, R₁ is selected from the group consisting of a substituted or unsubstituted (C6-C24) alkyl, (C6-C24) hydroxyalkyl, (C6-C24) alkyloxy, (C6-C24) alkylsulfo, (C6-C24) alkyloxy-(C6-C24) alkyl, (C6-C24) alkylsulfo-(C6-C24) alkyl, (C6-C24) alkylcarboxy-(C6-C24) alkyl, (C6-C24) alkylamino-(C6-C24) alkyl, (C6-C24) alkylamino-(C6-C24) alkylamino, (C6-C24) alkylamino-(C6-C24) alkylamino-(C6-C24) alkylamino, a substituted or unsubstituted (C6-C24) aminoalkyl, a substituted or unsubstituted aryl, a substituted or unsubstituted arylamino-(C6-C24) alkyl, (C6-C24) haloalkyl, C6-C24 alkenyl, C6-C24 alkynyl, and R₂ refers to a hydrogen or a methyl group having one, two, or three substituents, which may be the same or different, each replacing a hydrogen atom.

The term "substituted" in regard of R₂ used in formula (III) refers to a methyl group having one, two, or three substituents, which may be the same or different, each replacing a hydrogen atom. Examples of substituents include but are not limited to halogen (e.g., F, Cl, Br, or I), hydroxyl, protected hydroxyl, amino, protected amino, carboxy, protected carboxy, cyan, methylsulfonylamino, alkoxy, alkyl, aryl, arylalkyl, acyloxy, and lower haloalkyl.

An aliphatic group according to the invention is a branched or unbranched hydrocarbon that may be substituted or unsubstituted. Examples of branched alkyl groups include isopropyl, sec-butyl, isobutyl, tert-butyl, sec-pentyl, isopentyl, tert-pentyl, isohexyl. Substituted aliphatic groups may have one, two, three or more substituents, which may be the same or different, each replacing a hydrogen atom. Substituents are halogen (e.g., F, Cl, Br, or I), hydroxyl, protected hydroxyl, amino, protected amino, carboxy, protected carboxy, cyan, methylsulfonylamino, alkoxy, acyloxy, nitro, and lower haloalkyl.

Preferred lipophilic thiols falling under the general structure (I) that are suitable for the treatment of a malignant tumour disease or infectious disease are compounds in which R₁ + R₂ + R₃ + R₄ = C₆. Examples of such compounds are n-octylthiol or t-octylthiol. Examples in which R₁ + R₂ + R₃ + R₄ = C₁₀ are n-dodecylthiol, t-dodecylthiol and t-dodecylthiol isomer. Alternative variants of the inventive compounds in which R₁ + R₂ + R₃ + R₄ > C₁₀ are n-hexadecylthiol or n-octadecylthiol.

Preferred lipophilic trithiocarbonates falling under the general structure (II) that are suitable for the treatment of a malignant tumour disease or infectious disease are compounds in which R₁ = C₈ are S-octyl-S'[dimethyl-cyanomethyl]-trithiocarbonate, S-octyl-S'[methyl-hydroxypropyl-cyanomethyl]-trithiocarbonate, S-octyl-S'[methyl-carboxyethyl-cyanomethyl]-trithiocarbonate. Examples in which R₁ = C₁₂ are S-dodecyl-S'[dimethyl-cyanomethyl]-trithiocarbonate, S-dodecylS'[methyl-hydroxypropyl-cyanomethyl]-trithiocarbonate, S-dodecyl-S'[methyl-carboxyethyl-cyanomethyl]-trithiocarbonate.

Preferred lipophilic, aromatic dithioesters falling under the general structure (III) that are suitable for the treatment of a malignant tumour disease or infectious disease are compounds in which R₁ = C₁₂. Examples of compounds in which R₁ = C₁₂ are S-[dimethyl-cyanomethyl]-dodecylbenzodithioate, S-[methyl-hydroxypropyl-cyanomethyl]-dodecylbenzodithioate, or S-[methyl-carboxyethyl-cyanomethyl]-dodecylbenzodithioate. In alternative compounds, R₁ = O-C₁₂. Examples in which R₁ = O-C₁₂ are S-[dimethyl-cyanomethyl]-dodecanoxy-benzodithioate, S-[methyl-hydroxypropyl-cyanomethyl]-dodecanoxy-benzodithioate, or S-[methyl-carboxyethyl-cyanomethyl]- dodecanoxy-benzodithioate.

In an alternative embodiment relating to the general structure (IV) of the lipophilic, aromatic thiols, R₁ is selected from the group consisting of a substituted or unsubstituted (C6-C24) alkyl, (C6-C24) hydroxyalkyl, (C6-C24) alkyloxy, (C6-C24) alkylsulfo, (C6-C24) alkyloxy-(C6-C24) alkyl, (C6-C24) alkylsulfo-(C6-C24) alkyl, (C6-C24) alkylcarboxy-(C6-C24) alkyl, (C6-C24) alkylamino-(C6-C24) alkyl, (C6-C24) alkylamino-(C6-C24) alkylamino, (C6-C24) alkylamino-(C6-C24) alkylamino-(C6-C24) alkylamino, a substituted or unsubstituted (C6-C24) aminoalkyl, a substituted or unsubstituted aryl, a substituted or unsubstituted arylamino-(C6-C24) alkyl, (C6-C24) haloalkyl, C6-C24 alkenyl, C6-C24 alkynyl.

Preferred lipophilic, aromatic thiols comprising the general structure (IV) in which R₁ = C₁₂ or R₁ = X-C₁₂ are 4-dodecylthiophenol, O-dodecyl-4-hydroxythiophenol, S-dodecyl-1,4-benzenedithiol, or N-dodecyl-4-aminothiophenol. Preferred examples of compounds in which R₁ = C₁₈ or R₁ = X-C₁₈ are 4-octadecylthiophenol, O-octadecyl-4-hydroxythiophenol, S-octadecyl-1,4-benzenedithiol, or N-octadecyl-4-aminothiophenol.

The inventive compounds falling under the general formulas (I), (II), (III), or (IV) can be used for a variety of infectious diseases, preferably for the treatment of parasitic diseases that are caused by a number of parasites, including both single cell parasites and parasitic animals. Preferably, the parasitic infection is caused by parasites such as Acanthamoeba, Anisakis, Ascaris lumbricoides, Botfly, Balantidium coli, Bedbug, Cestoda (tapeworm), Chiggers, Cochliomyia hominivorax, Entamoeba histolytica, Fasciola hepatica, Giardia lamblia, Hookworm, Leishmania spp., Linguatula serrata, Liver fluke, Loa, Paragonimus - lung fluke, Pinworm, Plasmodium spp., Schistosoma spp., Strongyloides stercoralis, Mite, Tapeworm, Toxoplasma gondii, Trypanosoma spp., Whipworm, or Wuchereria bancrofti. The invention, however, is not restricted to these particular parasites. Preferred parasites that can be treated by the present invention are Leishmania spp., Plasmodium spp., Schistosoma spp., Trypanosoma spp.

As apparent, the invention can be applied for the therapy or prevention of parasitic diseases in animals, in particular pets, farm animals or breeding animals. In a first aspect, the inventive compounds can be applied to any infected host animal that shows clinical symptoms or suffers from a parasitic disease. In a second aspect, the inventive compounds can also be applied to animals that serve as a carrier for the parasite, i.e. third organisms that transmit the parasites to the host, but that do not suffer from the disease. As such, the inventive compounds are suitable for an eradication of an infection. Examples of trematodiasis and nematodiasis diseases that may be successfully treated are paragonimiasis, fasciolopsiasis, clonorchiasis and opisthorchiasis, fascioliasis, angiostrongyliasis, schistosomiasis. Preferred parasites to be treated by the inventive compounds in animals are selected from Leishmania spp., Schistosoma spp., Trypanosoma spp.

Due to the specific cytostatic and cytotoxic potential, the inventive compounds are suitable for tumour therapy, in particular for the treatment of malignant tumours. Preferably, the malignant tumour disease is selected from the group consisting of breast cancer, leukemia, squamous cell cancer, small-cell lung cancer, non-small cell lung cancer, gastrointestinal cancer, pancreatic cancer, glioblastoma, cervical cancer, ovarian cancer, liver cancer, bladder cancer, hepatoma, colon cancer, colorectal cancer, endometrial carcinoma, salivary gland carcinoma, kidney cancer, liver cancer, prostate cancer, vulval cancer, thyroid cancer, hepatic carcinoma or head and neck cancer.

The substances falling under the general formula (I), (IV), (II), or (III) are preferred embodiments of the present invention. All substances have in common that they can build thiyl radicals or sulfur radicals in the cell membrane, which is the underlying unifying inventive concept of the compounds of the present invention. Preferably, the compounds contain sulfur and are lipophilic. They can be distinguished in the way how the thiyl radical is stabilised, which can be aliphatic (class I) or aromatic (class IV). They can also be distinguished by the sulfur end groups such as a TTC group (class II) or a DTE group (class III).

The invention is further explained in the following examples.

### DESCRIPTION OF THE DRAWINGS:

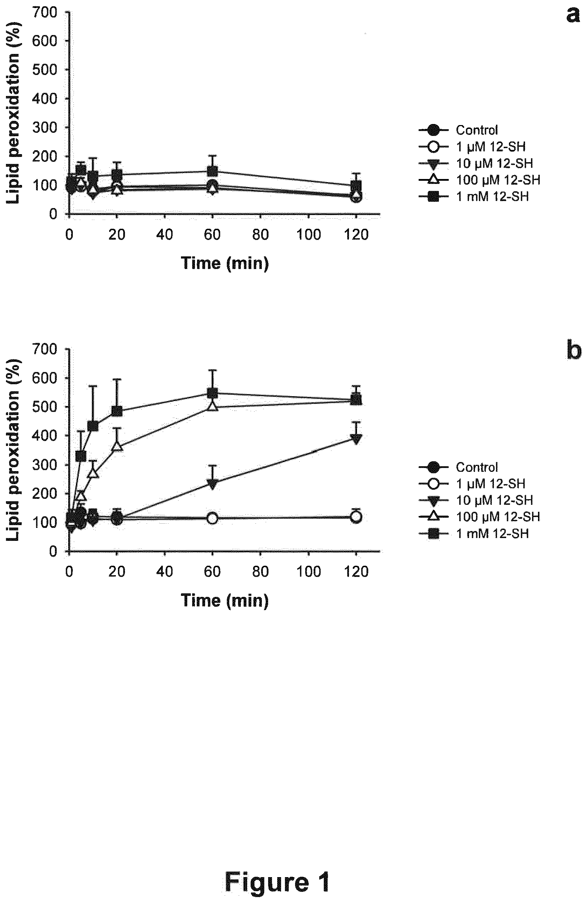

**Figure 1****. Prooxidative activity of chemical Chain-Transfer Agents (CTAs) in biological membranes.**
   Native lipid membranes from rat brain were examined on the extent of lipid peroxidation (as malondialdehyde) after treatment with the indicated concentrations of n-dodecylthiol (12-SH).
   Graph (a) shows the absence of any effect in the absence of any radical initiation ("dead membranes"); in graph (b), a low level of free radical initiator (here: iron/ascorbate) was added, as generally found in living cells depending on the cell type. The added amount of initiator alone did not yet have any measurable oxidizing effect (control in (b)); however, its hidden effect was massively amplified by the concomitant addition of a Chain-Transfer Agent.
   The data demonstrate the biochemical activity of the inventive compounds in biological membranes. As such they seem to act as initiator-dependent pro-oxidants.

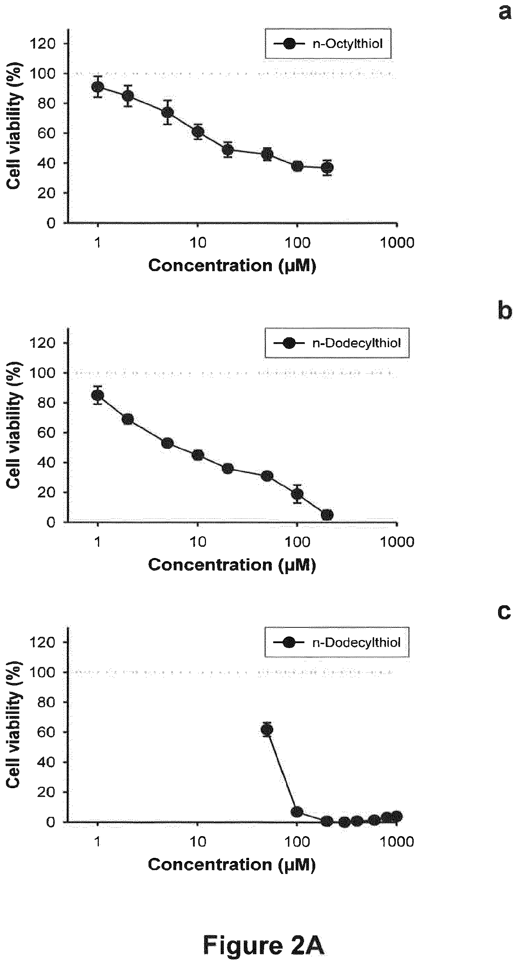

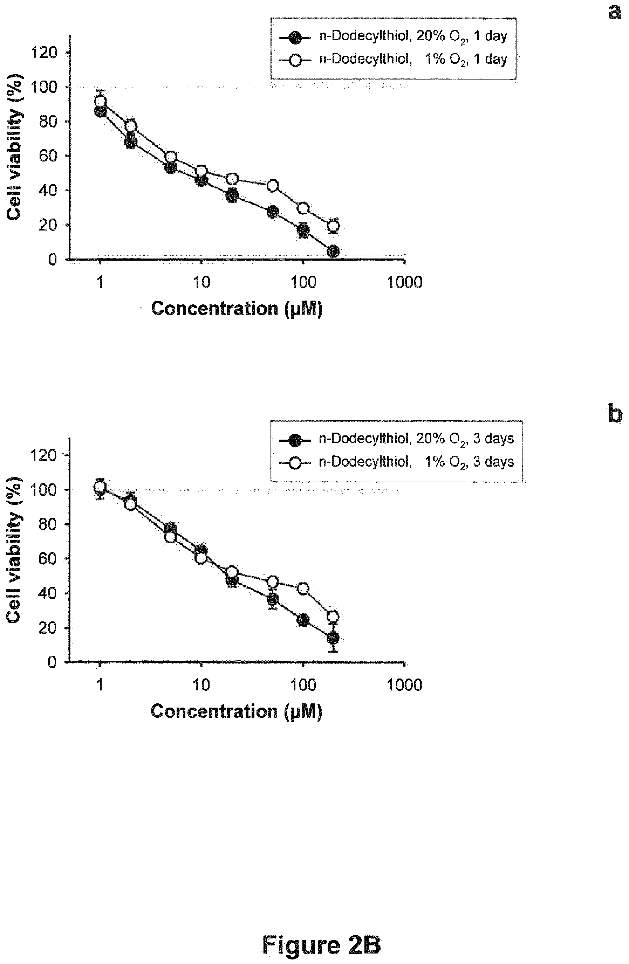

**Figure 2 (Figs. 2A to 2B****). Cytotoxic activity of Chain-Transfer Agents (CTAs) in cultivated HT22 cells and under hypoxic conditions.**
   The data confirm the basic toxicity of the compounds pursuant to the invention in living (tumour) cells. It is also shown that despite the postulated pro-oxidative mechanism of action, this toxicity is not dependent on the oxygen partial pressure within the (patho)physiological range of 1% to 20% oxygen, which is relevant for its potential use in hypoxic tumours.
**Figure 2A****. Cytotoxic activity of Chain-Transfer Agents (CTAs) in cultivated HT22 cells.**
   HT22 cells were treated for 3 days with the indicated concentrations of n-octylthiol (a) or n-dodecylthiol (b, c). The dotted line indicates the survival of control cultures treated with vehicle.
**Figure 2B****. Cytotoxic activity of Chain-Transfer Agents (CTAs) in cultivated HT22 cells under hypoxic conditions.**
   HT22 cells were treated for 1 day (a) or 3 days (b) with the indicated concentrations of n-dodecylthiol under normal oxygen conditions (20% O₂) or under highly hypoxic conditions (1% O₂). Such hypoxic conditions may prevail in solid tumours and can complicate treatment. However, the effectiveness of the chain-transfer agents was not compromised by hypoxic conditions.

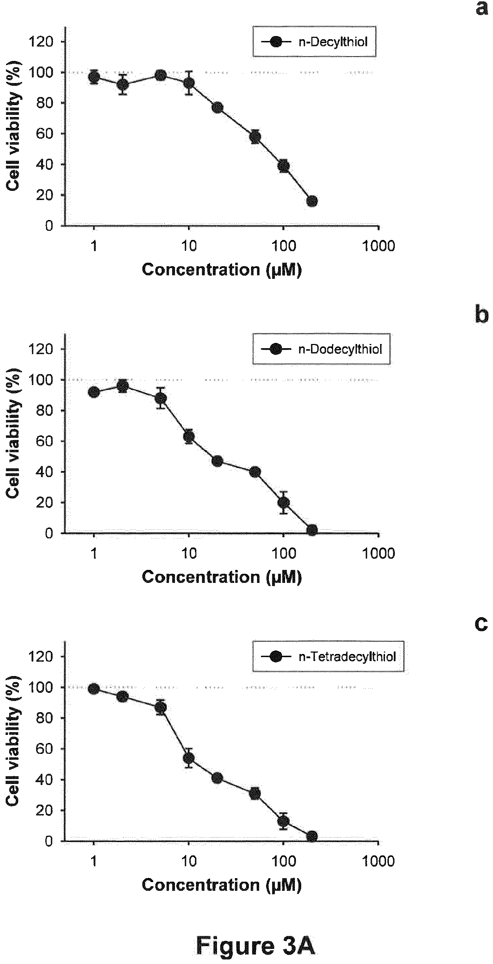

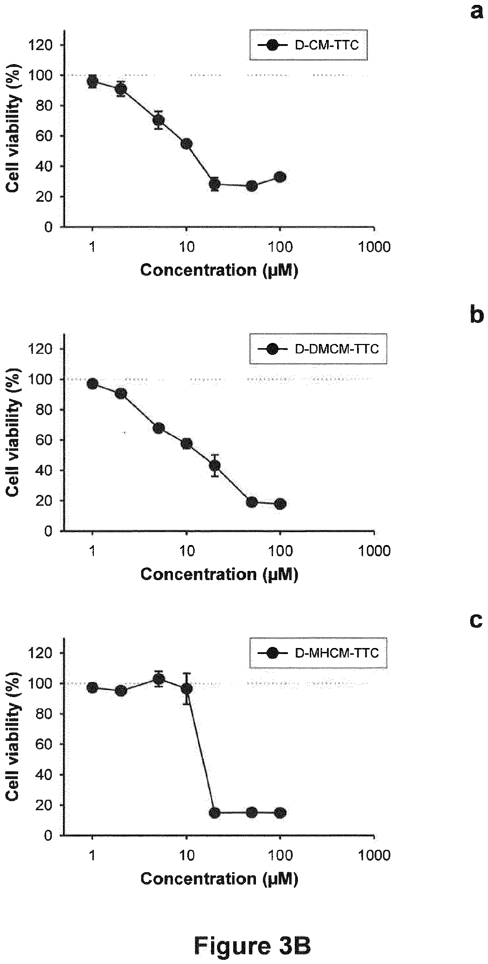

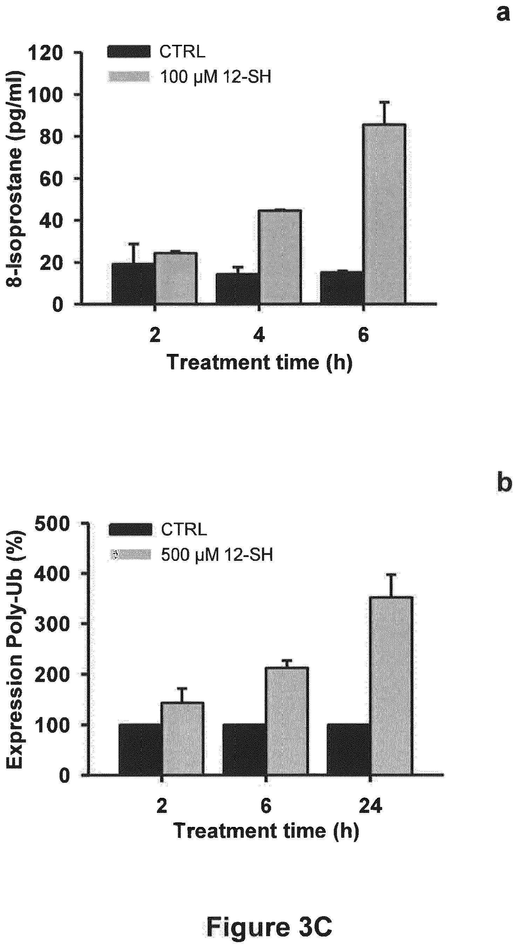

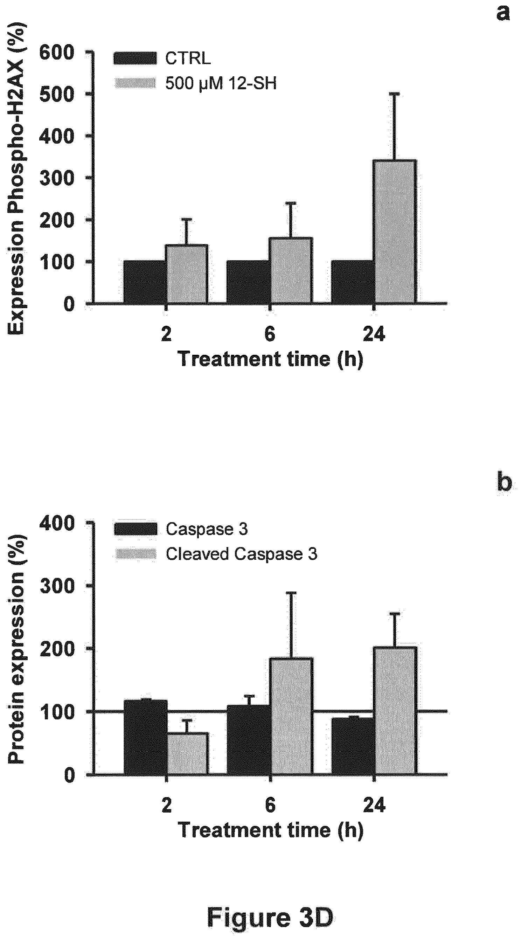

**Figure 3 (Figs. 3A to 3D****) exemplifies the activities of Chain-Transfer Agents (CTAs) in cultivated diploid human fibroblasts.**
   The data recapitulate the data from HT22 cells in normal fibroblasts. Representatives of lipophilic thiols falling under the general structure (I) and lipophilic trithiocarbonates falling under the general structure (II) have been used for experimentation. Furthermore, various analyses of the basic mechanism of action of the Chain-Transfer Agents in living cells were conducted. This includes analyses in regard of cellular lipid, protein and DNA damage as well as the physiological response of the cell to this damage. The observed types of damage (e.g. an induction of DNA double-strand breaks) indicate a particular efficacy of Chain-Transfer Agents in tumour cells.
**Figure 3A****. Activities of Chain-Transfer Agents (CTAs) in cultivated diploid human fibroblasts. Cytotoxicity of lipophilic thiols.**
   Primary human fibroblasts under cell division-stimulating culture conditions (medium with 10% fetal calf serum) were treated for 3 days with the indicated concentrations of n-decylthiol (a), n-dodecylthiol (b), or n-tetradecylthiol (c).
**Figure 3B****. Activities of Chain-Transfer Agents (CTAs) in cultivated diploid human fibroblasts. Cytotoxicity of lipophilic trithiocarbonates (TTC).**
   Primary human fibroblasts under cell division-stimulating culture conditions (medium with 10% fetal calf serum) were incubated for 3 days with the indicated concentrations of S-dodecyl-S'-cyanomethyl trithiocarbonate (D-CM-TTC) (a), S-dodecyl-S'-[dimethyl-cyanomethyl] trithiocarbonate (D-DMCM-TTC) (b), or S-dodecyl-S'-[methyl-hydroxypropyl cyanomethyl] trithiocarbonate (D-MHCM-TTC) (c). The dotted line indicates the survival of control cultures treated with vehicle.
**Figure 3C****. Activities of Chain-Transfer Agents (CTAs) in cultivated diploid human fibroblasts. Biochemical effects.**
   Primary human fibroblasts under cell division-stimulating culture conditions (medium with 10% fetal calf serum) were treated with 100 µM (a) and 500 µM (b) n-dodecylthiol (12-SH), respectively, over the indicated time period. Graph (a) shows the concentration of released lipid peroxidation marker 8-isoprostane, graph (b) shows the amount of polyubiquitinated proteins in the cell, which are a marker of protein damage. Both pro-oxidative markers increased significantly within a few hours.
**Figure 3D****. Activities of Chain-Transfer Agents (CTAs) in cultivated diploid human fibroblasts. Genotoxic and pro-apoptotic effects.**
   Primary human fibroblasts under cell division-stimulating culture conditions (medium with 10% fetal calf serum) were treated with 500 µM n-dodecylthiol (12-SH) for the indicated time. Panel (a) shows the protein expression of Ser-140 phosphorylated histone 2AX, a marker for DNA double strand breaks and general laboratory proxy for DNA damage. Graph (b) shows the expression of caspase 3 and its active fragment "cleaved" caspase 3. Cleaved caspase 3 is the most important executing caspase in the apoptosis cascade.

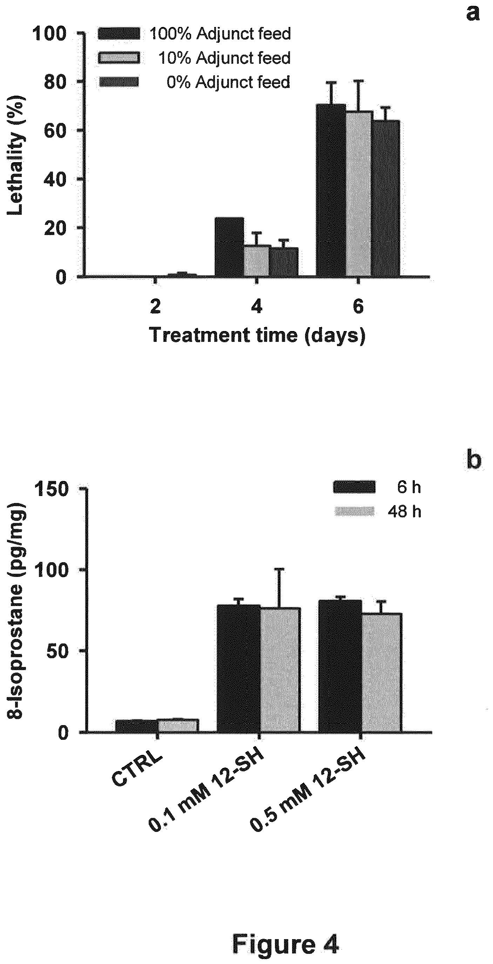

**Figure 4****. Prooxidative toxicity of chemical Chain-Transfer Agents (CTAs) in nematodes in vivo.**
   Nematodes (C. elegans) were treated with 0.5 mM n-dodecylthiol in the minimum diet supplemented with varying amounts of coliform bacteria as adjunct feed. At the times indicated, the number of worms killed (a) was counted. Panel (b) shows the concentration of the lipid peroxidation marker 8-isoprostane in homogenates of worms treated with 0.1 mM or 0.5 mM n-dodecylthiol (12-SH). This biochemical marker of toxicity increased to a constant maximum after only 6 h, which then led to death after a few days (a).
   The data confirm the general cytotoxic activity of the substances according to the invention in vivo, after oral administration. Also, their pro-oxidative mechanism of action in vivo is evidenced biochemically.

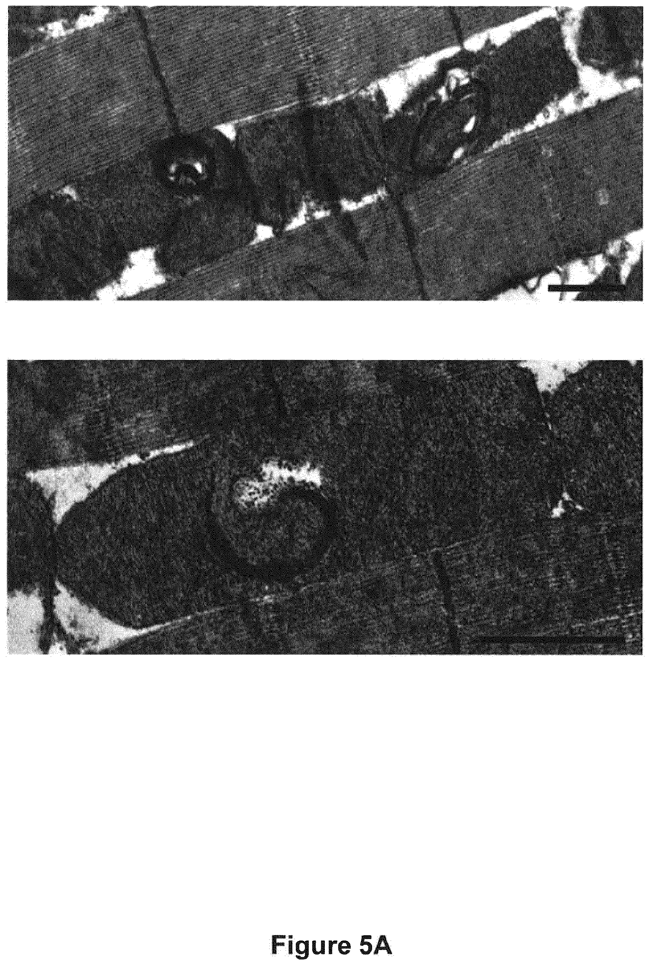

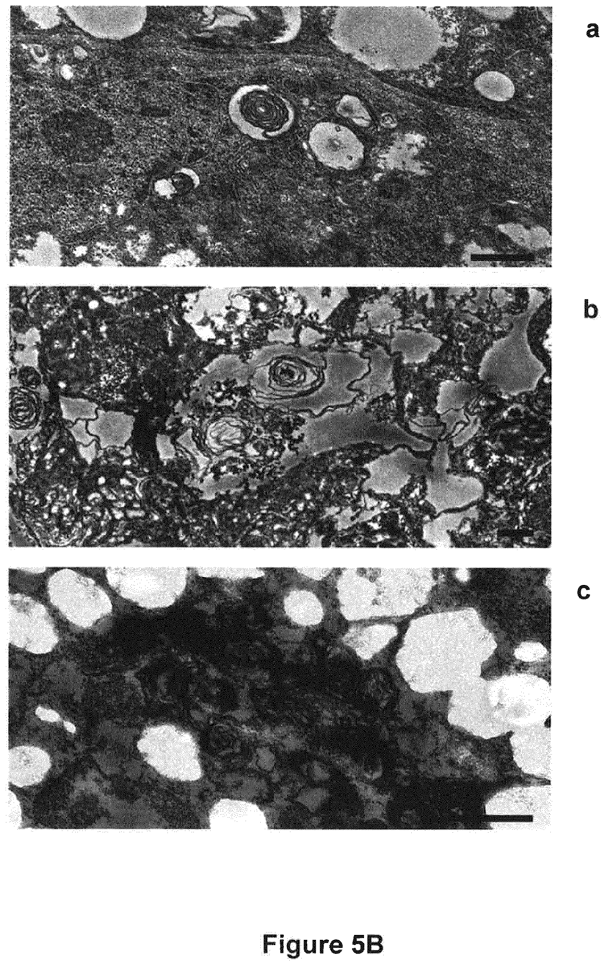

**Figure 5 (Figs. 5A to 5B****). Ultrastructural effects of Chain-Transfer Agents (CTAs) in insects on different organs in vivo.**
   The data demonstrate the effectiveness of chain-transfer agents in vivo in a second, higher organism, in fruit flies (Drosophila). They also show directly the predicted mitochondrial specificity of the cytotoxic effect of these agents.
**Figure 5A****. Ultrastructural effects of Chain-Transfer Agents (CTAs) in insects in vivo. Muscle mitochondria.**
   Fruit flies were treated with 1 mM n-dodecylthiol in the feed. The two electron micrographs show intramitochondrial, spiral multilamellar lesions. In tumour cells, such lesions would be expected to lead to apoptosis. The myofibrillar structure, on the other hand, is intact. The black line corresponds to approximately 1 µm.
**Figure 5B****. Ultrastructural effects of Chain-Transfer Agents (CTAs) in insects in vivo. Nervous system and eye.**
   Fruit flies were treated with 1 mM n-dodecylthiol in the feed. The electron micrographs show spiral multilamellar lesions of mitochondrial origin in the nervous system (a, b) and in photoreceptor cells (c). Other damage phenotypes are the vacuolization and electron-dense aggregation in (b) as well as the bright lipofuscin accumulation in (c). The black line corresponds to approximately 1 µm.

### MATERIAL AND METHODS:

### Prooxidative activity of chemical Chain-Transfer Agents (CTAs) in biological membranes (Figure 1).

Native biological membranes from adult rat brain were prepared by differential centrifugation as described (Moosmann and Behl, Proc Natl Acad Sci USA 96:8867-8872, 1999). Samples containing 0.5 mg/ml total protein (as per bicinchoninic acid assay from Pierce, Rockford, IL, USA) were solubilized by brief sonication in PBS (phosphate-buffered saline) and administered with the indicated concentrations of n-dodecyl thiol (12-SH; from Sigma-Aldrich, St. Louis, MO, USA) dissolved in ethanol (final concentration: 0.1%). Subsequently, 10 µM Fe²⁺/200 µM ascorbate were added as radical-initiating mix; controls received vehicle (water). After the indicated time, the reaction was stopped by adding 2.5 volumes of 5% trichloroacetic acid in 1 M acetic acid, followed by centrifugation (10,000 g for 10 min). Subsequently, thiobarbituric acid-reactive substances (TBARS) as marker of lipid peroxidation were quantified fluorimetrically as detailed before (Hajieva et al., J Neurochem 110:118-132, 2009).

### Cytotoxic activity of Chain-Transfer Agents (CTAs) in cultivated HT22 cells (Figure 2).

**(****Fig. 2A****)** HT22 are a widely used, immortalized neuronal cell line that has been generated by transforming mouse neuronal tissue with the oncogenic SV40 T-antigen (Morimoto and Koshland, Neuron 5:875-880, 1990). HT22 cells were cultivated in high-glucose DMEM (Dulbecco's Modified Eagle's Medium) supplemented with 10% heat-inactivated FCS (fetal calf serum), 1 mM pyruvate, 100 mg/L streptomycin and 100 U/mL penicillin. For routine culture, cells were grown in 100 mm dishes at 37°C in a humidified atmosphere containing 5% CO₂. They were passaged three times per week on reaching approximately 90% confluence and splitted 1:10. All cell culture reagents were from Invitrogen, Carlsbad, CA, USA.

For cell survival experiments, HT22 cells were seeded into 96-well plates at a density of ∼ 5000 cells per well in 0.1 mL medium. After 24 h cultivation, the cells were administered with the indicated concentrations of the tested compounds (n-octyl thiol (8-SH) or n-dodecyl thiol (12-SH)) dissolved in ethanol (final concentration: 1%). After 1 day or 3 days of incubation as indicated, cell survival was analyzed by colorimetric MTT reduction tests (MTT is 3-(4,5-dimethylthiazol-2-yl-)-2,5 diphenyltetrazolium bromide) which were performed exactly as described (Hajieva et al., J Neurochem 110:118-132, 2009).

**(****Fig. 2B****.)** Experiments under hypoxic conditions were conducted accordingly, with the only difference that the 1 day- or 3 day-incubation after n-dodecyl thiol administration was done in a separate incubator that had been set to contain only 1% oxygen (through the controlled addition of nitrogen). The fraction of CO₂ was maintained at 5%.

### Activities of Chain-Transfer Agents (CTAs) in cultivated diploid human fibroblasts (Figure 3).

**(****Fig. 3A****)** Normal human diploid fibroblasts were cultivated under standard conditions stimulating cell division, i.e. with 10% serum in the medium. Specifically, the cells were grown in high-glucose DMEM (Dulbecco's Modified Eagle's Medium) supplemented with 10% heat-inactivated FCS (fetal calf serum), 1x non-essential amino acid (NEAA) supplement, 1 mM pyruvate, 100 mg/L streptomycin and 100 U/mL penicillin. For cultivation, the cells were maintained at 37°C in a humidified atmosphere containing 5% CO₂. The cells were generally passaged on reaching approximately 90% confluence and were seeded, after counting of the PDLs (population doublings), in new dishes at a density of 10⁶ cells per dish in 100 mm dishes, or at a density of 10⁴ cells per well in 96-well plates. Cells were used for experiments at PDL 25-30. All cell culture reagents were from Invitrogen, Carlsbad, CA, USA.

For cell survival experiments, cells cultivated in 96-well plates for 24 h were administered with the indicated concentrations of the tested compounds (n-decyl thiol (10-SH), n-dodecyl thiol (12-SH), n-tetradecyl thiol (14-SH)) dissolved in ethanol (final concentration: 1%). After 3 days of incubation, cell survival was analyzed by colorimetric MTT reduction tests (MTT is 3-(4,5-dimethylthiazol-2-yl-)-2,5 diphenyltetrazolium bromide) which were performed exactly as described (Hajieva et al., J Neurochem 110:118-132, 2009).

**(****Fig. 3B****)** Cells cultivated in 96-well plates for 24 h were administered with the indicated concentrations of the tested compounds (S-dodecyl-S'-cyanomethyl-trithiocarbonate (D-CM-TTC), S-dodecyl-S'-[dimethyl-cyanomethyl]-trithiocarbonate (D-DMCM-TTC), or S-dodecyl-S'-[methyl-hydroxypropyl-cyanomethyl]-trithiocarbonate (D-MHCM-TTC)) dissolved in ethanol (final concentration: 1%). After 3 days of incubation, cell survival was analyzed by colorimetric MTT reduction tests as above.

**(****Fig. 3C****)** 8-Isoprostanes. Cells cultivated in 96-well plates for 24 h were administered with 100 µM n-dodecyl thiol (12-SH) for the indicated time before removing the cell culture supernatant for 8-isoprostane analysis. 8-Isoprostane analysis was achieved by a commercial enzyme immunoassay (Cayman Chemicals, Ann Arbor, MI, USA), which was conducted as detailed in the manufacturer's instructions.

Protein polyubiquitination. Cells cultivated in 100 mm dishes for 24 h were administered with 500 µM n-dodecyl thiol (12-SH) for the indicated time before harvesting of the cells in lysis buffer (50 mM Tris-HCI, pH 7.4, 10% sucrose, 1 mM EDTA, 1 mM EGTA, 1 mM Na₃VO₄, 1 mM NaF, 1x protease inhibitor cocktail from Sigma-Aldrich, St. Louis, MO, USA) followed by brief sonication and denaturation at 95°C for 2 min. For the specific analysis of protein ubiquitination, Western immunoblotting was performed as described (Hajieva et al., J Neurochem 110:118-132, 2009). In brief, equal amounts of total protein (as per bicinchoninic acid assay from Pierce, Rockford, IL, USA) were separated by 12% SDS-polyacrylamide gel electrophoresis (PAGE) and transferred onto nitrocellulose membranes by electroblotting. Blocking of was carried out by incubation with Tris-buffered saline/Tween-20 (TBST) containing 2% fat-free dry milk for 60 min at 20°C, followed by incubation with the specific primary antibodies at 4°C overnight. The primary antibodies were: Rabbit anti-polyubiquitin antibody (dilution 1:2000; from Agilent Dako, Santa Clara, CA, USA); mouse anti-α-tubulin antibody (dilution 1:1000; from Sigma-Aldrich, St. Louis, MO, USA); both diluted in TBST. The next day, the membranes were treated with horseradish peroxidase-conjugated secondary anti-mouse or anti-rabbit antibodies (1:5000; from Jackson Immunoresearch, West Grove, PA, USA) for 90 min at 20°C. Subsequently, the membranes were washed 3x 15 min with TBST. Immunoreactive bands were developed using commercial peroxidase substrate kits (Enhanced Chemiluminescence Plus from Amersham Pharmacia Biotech, Piscataway, NJ, USA), and scanned with a digital chemiluminescent imaging system. Densitometric quantification was performed using automated image analysis software.

**(****Fig. 3D****)** Phospho-H2AX expression and caspase 3 expression. Cells cultivated in 100 mm dishes for 24 h were administered with 500 µM n-dodecyl thiol (12-SH) for the indicated time before harvesting of the cells in lysis buffer and processing for Western blotting as above (Hajieva et al., J Neurochem 110:118-132, 2009). The primary antibodies were: Rabbit anti-phospho-histone H2AX (Ser139) antibody (dilution 1:1000; from Cell Signaling, Cambridge, UK); rabbit anti-caspase 3 antibody (dilution 1:1000; from Cell Signaling, Cambridge, UK); mouse anti-α-tubulin antibody (dilution 1:1000; from Sigma-Aldrich, St. Louis, MO, USA); all diluted in TBST.

### Prooxidative toxicity of chemical Chain-Transfer Agents (CTAs) in nematodes in vivo (Figure 4).

Caenorhabditis elegans N2 Bristol strain animals were expanded and cultivated at 20°C on nematode growth medium (NGM) plates in the presence of Escherichia coli strain HB101 as food source following standard protocols (Mocko et al., Neurobiol Dis 40:120-129, 2010). For the toxicity experiments, synchronized adult worms aged 2 days were maintained in liquid culture medium (S-Basal medium supplemented with 5 mg/L cholesterol, 100 mg/L streptomycin, 100 mg/L fluorodeoxyuridine (FUDR)) to which varying amounts of Escherichia coli were added (measured as optical density (OD) at 600 nm wavelength) as described (Mair et al., PLoS One 4:e4535, 2009). Worms distributed in 48-well plates were administered with 500 µM n-dodecyl thiol (12-SH) dissolved in ethanol (final concentration: 1%) and analyzed, after the indicated time, for survival by visual inspection and mechanical stimulation (nose-touch assay) as detailed (Mocko et al., Neurobiol Dis 40:120-129, 2010).

8-Isoprostanes. Synchronized, 4-day-old adult worms distributed in cell culture flasks were administered with the indicated concentration of n-dodecyl thiol (12-SH) dissolved in ethanol (final concentration: 1%) and cultivated for 6 h or 48 has indicated. The worms were collected by centrifugation at 1200 g, washed twice with S-Basal medium, and once with DMEM medium containing 100 µM butylated hydroxytoluene (BHT). The worms were then homogenized in DMEM/BHT by sonication (3x 20s at 30 kHz on ice). Equal amounts of protein of the resulting homogenate (as per bicinchoninic acid assay from Pierce, Rockford, IL, USA) were probed for the presence of 8-isoprostane by a commercial enzyme immunoassay (Cayman Chemicals, Ann Arbor, MI, USA) following the manufacturer's instructions.

### Ultrastructural effects of Chain-Transfer Agents (CTAs) in insects in vivo (Figure 5).

Male Drosophila melanogaster (strain Oregon-R) were maintained at 25°C in plastic vials covered with air-permeable lids and received standard food (50 g/L refined household sugar, 50 g/L baker's yeast, and 20 g/L agar powder). The medium was boiled under stirring, adding the following supplements at approximately 70°C: 3 g/L methylparabene (dissolved in ethanol, final concentration: 0.15%), 3 mL/L propionic acid and the appropriate amount of n-dodecyl thiol (12-SH) dissolved in ethanol (final concentration: 0.1%). Synchronized male flies were transferred into new vials and scored for survival every other day. Treatment started on day 2 of adulthood.

Electron microscopy. Flies harvested after 50 days of treatment were cryofixed by plunge freezing, cut into head, thorax and abdomen before chemical fixation for 90 min with 3% glutaraldehyde and 3% formaldehyde in PBS. The tissues were washed, fixed with 2% OsO₄, washed again, dehydrated with rising concentrations of ethanol, transitionally stabilized with propylene oxide and embedded into epoxy resin essentially as described (Bozzola and Russel, Electron Microscopy: Jones and Bartlett Publishers, Inc., Sudbury, MA, 1999). The polymerized blocks were mounted, trimmed, and sectioned in an ultramicrotome before transfer onto electron microscopy grids. Images were acquired under standard conditions in a Tecnai Transmission Electron Microscope (FEI Company, Hillsboro, OR, USA).

### NON-PATENT LITERATURE:

Aktories K, Förstermann U, Hofmann FB, Starke K, Editoren (2017). Allgemeine und spezielle Pharmakologie und Toxikologie: Begründet von W. Förth, D. Henschler, W. Rummel. Elsevier Health Sciences, Amsterdam.
Albonico M, Levecke B, LoVerde PT, Montresor A, Prichard R, Vercruysse J, Webster JP (2015). Monitoring the efficacy of drugs for neglected tropical diseases controlled by preventive chemotherapy. J Glob Antimicrob Resist 3, 229-236.
Barker HE, Paget JT, Khan AA, Harrington KJ (2015). The tumour microenvironment after radiotherapy: mechanisms of resistance and recurrence. Nat Rev Cancer 15, 409-425.
Brown JM, Wilson WR (2004). Exploiting tumour hypoxia in cancer treatment. Nat Rev Cancer 4, 437-447.
Castro JA, de Mecca M, Bartel LC (2006). Toxic side effects of drugs used to treat Chagas1 disease (American trypanosomiasis). Hum Exp Toxicol 25, 471-479.
Collins I. and Workman P (2006). New approaches to molecular cancer therapeutics. Nat Chem Biol, 2 (12): 689-700.
Cui Q, Wen S, Huang P (2017). Targeting cancer cell mitochondria as a therapeutic approach: recent Updates. Future Med Chem 9, 929-949.
Dolgin E (2018). Bringing down the cost of cancer treatment. Nature 555, S26-S29.
Dolmans DE, Fukumura D, Jain RK (2003). Photodynamic therapy for cancer. Nat Rev Cancer 3, 380-387.
Donaldson G. P., Lee S. M., Mazmanian S. K. (2016). Gut biogeography of the bacterial microbiota. Nat. Rev. Microbiol. 14, 20-32.
Eijck I. A., Borgsteede F. H. (2005). A survey of gastrointestinal pig parasites on free-range, organic and conventional pig farms in The Netherlands. Vet. Res. Commun. 29, 407-414.
Frézal L., Félix M.-A. (2015). C. elegans outside the Petri dish; eLife. 4: e05849.
Galadari S, Rahman A, Pallichankandy S, Thayyullathil F (2017). Reactive oxygen species and cancer paradox: To promote or to suppress? Free Radic Biol Med 104,144-164.
Gorrini C, Harris IS, Mak TW (2013). Modulation of oxidative stress as an anticancer strategy. Nat Rev Drug Discov 12, 931-947.
Greenwood B (2014). Treatment of malaria- a continuing challenge. N Engl J Med 371, 474-475.
Hotez P. J., Brindley P. J., Bethony J. M., King C. H., Pearce E. J., Jacobson J. (2008). Helminth infections: the great neglected tropical diseases. J. Clin. Invest. 118, 1311-1321.
Kavishe RA, Koenderink JB, Alifrangis M (2017). Oxidative stress in malaria and artemisinin combination therapy: Pros and Cons. FEBS J 284, 2579-2591.
Krücken J, Fraundorfer K, Mugisha JC, Ramünke S, Sifft KC, Geus D, Habarugira F, Ndoli J, Sendegeya A,Mukampunga C, Bayingana C, Aebischer T, Demeler J, Gahutu JB, Mockenhaupt FP, von Samson-Himmelstjerna G (2017) Reduced efficacy of albendazole against Ascaris lumbricoides in Rwandan schoolchildren. Int J Parasitol Drugs Drug Resist 7, 262-271.
Lanusse C, Canton C, Virkel G, Alvarez L, Costa-Junior L, Lifschitz A (2018). Strategies to optimize the efficacy of anthelmintic drugs in ruminants. Trends Parasitol 34, 664-682.
Li W, Mo W, Shen D, Sun L, Wang J, Lu S, Gitschier JM, Zhou B (2005). Yeast model uncovers dual roles of mitochondria in action of artemisinin. PLoS Genet 1, e36.
Lipton SA (2007). Pathologically activated therapeutics for neuroprotection. Nat Rev Neurosci 8, 803-808.
Löfmark S, Edlund C, Nord CE (2010). Metronidazole is still the drug of choice for treatment of anaerobic infections. Clin Infect Dis 50 Suppl 1, S16-23.
Mehlotra RK (1996). Antioxidant defense mechanisms in parasitic protozoa. Crit Rev Microbiol 22, 295-314.
Molyneux DH, Savioli L, Engels D (2017). Neglected tropical diseases: progress towards addressing the chronic pandemic. Lancet 389, 312-325.
Moss RW (2007). Do antioxidants interfere with radiation therapy for cancer? Integr Cancer Ther 6, 281-292.
Nganga C. J., Karanja D. N., Mutune M. N. (2008). The prevalence of gastrointestinal helminth infections in pigs in Kenya. Trop. Anim. Health Prod. 40, 331-334.
Pardee AB, Stein, GS, editors (2009). The biology and treatment of cancer: Understanding cancer. John Wiley & Sons, New York.
Rosso M. N., Pujol N., Ewbank J. J. (2013). Innate immunity in Caenorhabditis elegans and other nematodes, in Parasitic Nematodes: Molecular Biology, Biochemistry and Immunology, eds Kennedy M. W., Harnett W., editors. (Wallingford: CABI;), 42-66.
Sessa C, Gianni L, Garassino M, van Halteren H (2012). ESMO Handbook of clinical pharmacology of anticancer agents. European Society for Medical Oncology (ESMO), Viganello-Lugano.
Siegel RL, Miller KD, Jemal A (2019). Cancer statistics, CA Cancer J Clin 69, 7-34.
Sosa V, Moline T, Somoza R, Paciucci R, Kondoh H, LLeonart ME (2013). Oxidative stress and cancer: an overview. Ageing Res Rev 12, 376-390.
Szatrowski TP, Nathan CF (1991). Production of large amounts of hydrogen peroxide by human tumor cells. Cancer Res 51, 794-798.
Tarr D. E. K. (2012). Distribution and characteristics of ABFs, cecropins, nemapores, and lysozymes in nematodes. Dev. Comp. Immunol. 36, 502-520.
Toler SM, Noe D, Sharma A (2006). Selective enhancement of cellular oxidative stress by chloroquine: implications for the treatment of glioblastoma multiforme. Neurosurg Focus 21, E10.
Trachootham D, Alexandre J, Huang P (2009). Targeting cancer cells by ROS-mediated mechanisms: a radical therapeutic approach? Nat Rev Drug Discov 8, 579-591.
Tse EG, Korsik M, Todd MH (2019). The past, present and future of anti-malarial medicines. Malar J 18, 93.
Turrens JF (2004). Oxidative stress and antioxidant defenses: a target for the treatment of diseases caused by parasitic protozoa. Mol Aspects Med 25, 211-220.
World Health Organization (2017). Fact Sheet: Soil-Transmitted Helminth Infections.

## Claims

1. A prooxidative chain-transfer agent selected from the group consisting of
(1) lipophilic thiols comprising the general structure (I) 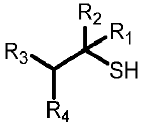 wherein R₁ - R₄ are hydrogen or aliphatic or mixed aliphatic-aromatic or mixed aliphatic-heteroatom groups in which the number of aliphatic carbon atoms is at least six: R₁ + R₂ + R₃ + R₄ ≥ C₆;
(2) lipophilic trithiocarbonates comprising the general structure (II) 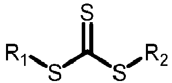
wherein R₁ is an aliphatic or mixed aliphatic-aromatic or mixed aliphatic-heteroatom group in which the number of aliphatic carbon atoms is at least six: R₁ ≥ C₆;
R₂ is a hydrogen or a methyl group or a substituted methyl group;
(3) lipophilic, aromatic dithioesters comprising the general structure (III) 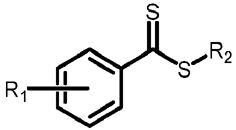
wherein R₁ is an aliphatic or mixed aliphatic-aromatic or mixed aliphatic-heteroatom group in which the number of aliphatic carbon atoms is at least six: R₁ ≥ C₆;
R₂ is a hydrogen or a methyl group or a substituted methyl group;
(4) lipophilic, aromatic thiols comprising the general structure (IV) 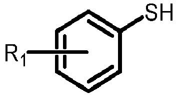 wherein R₁ is an aliphatic or mixed aliphatic-aromatic or mixed aliphatic-heteroatom group in which the number of aliphatic carbon atoms is at least six: R₁ ≥ C₆,
for use in the treatment of a malignant tumour disease, or infectious disease.

2. The prooxidative chain-transfer agent according to claim 1, wherein in the general structure (I) of the lipophilic thiols the hydrophobic group is branched, substituted or unsubstituted.

3. The prooxidative chain-transfer agent according to claim 1, wherein in the general structure (I) of the lipophilic thiols each R₁ - R₄ substituent comprises a hydrophobic group comprising one or more S, O or N heteroatoms.

4. The prooxidative chain-transfer agent according to claim 1, wherein in the general structure (I) of the lipophilic thiols each R1 - R4 is selected from the group consisting of hydrogen, hydroxyl, a substituted or unsubstituted (C1-C24) alkyl, (C1-C24) hydroxyalkyl, (C1-C24) alkyloxy-(C1-C24) alkyl, (C1-C24) alkylsulfo-(C1-C24) alkyl, (C1-C24) alkylcarboxy-(C1-C24) alkyl, (C1-C24) alkylamino-(C1-C24)alkyl, (C1-C24) alkylamino-(C1-C24) alkylamino, (C1-C24) alkylamino-(C1-C24) alkylamino-(C1-C24) alkylamino, a substituted or unsubstituted (C1-C24) aminoalkyl, a substituted or unsubstituted aryl, a substituted or unsubstituted arylamino-(C1-C24) alkyl, (C1-C24) haloalkyl, C2-C24 alkenyl, C2-C24 alkynyl, oxo, sulfo.

5. The prooxidative chain-transfer agent according to claim 1, wherein in the general structure (II) of the lipophilic trithiocarbonates R1 is selected from the group consisting of a substituted or unsubstituted (C6-C24) alkyl, (C6-C24) hydroxyalkyl, (C6-C24) alkyloxy, (C6-C24) alkylsulfo, (C6-C24) alkyloxy-(C6-C24) alkyl, (C6-C24) alkylsulfo-(C6-C24) alkyl, (C6-C24) alkylcarboxy-(C6-C24) alkyl, (C6-C24) alkylamino-(C6-C24) alkyl, (C6-C24) alkylamino-(C6-C24) alkylamino, (C6-C24) alkylamino-(C6-C24) alkylamino-(C6-C24) alkylamino, a substituted or unsubstituted (C6-C24) aminoalkyl, a substituted or unsubstituted aryl, a substituted or unsubstituted arylamino-(C6-C24) alkyl, (C6-C24) haloalkyl, C6-C24 alkenyl, C6-C24 alkynyl, and R₂ refers to a hydrogen or a methyl group having one, two, or three substituents, which may be the same or different, each replacing a hydrogen atom.

6. The prooxidative chain-transfer agent according to claim 5, wherein in the general structure (II) of the lipophilic trithiocarbonates the substitutent is selected from the group consisting of a halogen, hydroxyl, protected hydroxyl, amino, protected amino, carboxy, protected carboxy, cyan, methylsulfonylamino, alkoxy, alkyl, aryl, arylalkyl, acyloxy, or lower haloalkyl.

7. The prooxidative chain-transfer agent according to claim 1, wherein in the general structure (III) of the lipophilic, aromatic dithioesters R₁ is selected from the group consisting of a substituted or unsubstituted (C6-C24) alkyl, (C6-C24) hydroxyalkyl, (C6-C24) alkyloxy, (C6-C24) alkylsulfo, (C6-C24) alkyloxy-(C6-C24) alkyl, (C6-C24) alkylsulfo-(C6-C24) alkyl, (C6-C24) alkylcarboxy-(C6-C24) alkyl, (C6-C24) alkylamino-(C6-C24) alkyl, (C6-C24) alkylamino-(C6-C24) alkylamino, (C6-C24) alkylamino-(C6-C24) alkylamino-(C6-C24) alkylamino, a substituted or unsubstituted (C6-C24) aminoalkyl, a substituted or unsubstituted aryl, a substituted or unsubstituted arylamino-(C6-C24) alkyl, (C6-C24) haloalkyl, (C6-C24) alkenyl, (C6-C24) alkynyl, and R₂ refers to a hydrogen or a methyl group having one, two, or three substituents, which may be the same or different, each replacing a hydrogen atom.

8. The prooxidative chain-transfer agent according to claim 7, wherein in the general structure (III) of the lipophilic, aromatic dithioesters, the substituent is selected from the group consisting of a halogen, hydroxyl, protected hydroxyl, amino, protected amino, carboxy, protected carboxy, cyan, methylsulfonylamino, alkoxy, alkyl, aryl, arylalkyl, acyloxy, or lower haloalkyl.

9. The prooxidative chain-transfer agent according to claim 1, wherein the lipophilic thiol comprising the general structure (I) is selected from the group consisting of n-octylthiol, t-octylthiol, n-dodecylthiol, t-dodecylthiol, t-dodecylthiol isomer, n-hexadecylthiol, or n-octadecylthiol.

10. The prooxidative chain-transfer agent according to claim 1, wherein the lipophilic trithiocarbonate comprising the general structure (II) is selected from the group consisting of S-octyl-S'[dimethyl-cyanomethyl]-trithiocarbonate, S-octyl-S'[methyl-hydroxypropyl-cyanomethyl]-trithiocarbonate, S-octyl-S'[methyl-carboxyethyl-cyanomethyl]-trithiocarbonate, S-dodecyl-S'[dimethyl-cyanomethyl]-trithiocarbonate, S-dodecyl-S'[methyl-hydroxypropyl-cyanomethyl]-trithiocarbonate, or S-dodecyl-S'[methyl-carboxyethyl-cyanomethyl]-trithiocarbonate.

11. The prooxidative chain-transfer agent according to claim 1, wherein the lipophilic, aromatic dithioester comprising the general structure (III) is selected from the group consisting of S-[dimethyl-cyanomethyl]-dodecylbenzodithioate, S-[methyl-hydroxypropyl-cyanomethyl]-dodecylbenzodithioate, S-[methyl-carboxyethyl-cyanomethyl]-dodecylbenzodithioate, S-[dimethyl-cyanomethyl]-dodecanoxy-benzodithioate, S-[methyl-hydroxypropyl-cyanomethyl]- dodecanoxy-benzodithioate, or S-[methyl-carboxyethyl-cyanomethyl]- dodecanoxy-benzodithioate.

12. The prooxidative chain-transfer agent according to claim 1, wherein in the general structure (IV) of the lipophilic, aromatic thiols, R₁ is selected from the group consisting of a substituted or unsubstituted (C6-C24) alkyl, (C6-C24) hydroxyalkyl, (C6-C24) alkyloxy, (C6-C24) alkylsulfo, (C6-C24) alkyloxy-(C6-C24) alkyl, (C6-C24) alkylsulfo-(C6-C24) alkyl, (C6-C24) alkylcarboxy-(C6-C24) alkyl, (C6-C24) alkylamino-(C6-C24) alkyl, (C6-C24) alkylamino-(C6-C24) alkylamino, (C6-C24) alkylamino-(C6-C24) alkylamino-(C6-C24) alkylamino, a substituted or unsubstituted (C6-C24) aminoalkyl, a substituted or unsubstituted aryl, a substituted or unsubstituted arylamino-(C6-C24) alkyl, (C6-C24) haloalkyl, (C6-C24) alkenyl, (C6-C24) alkynyl.

13. The prooxidative chain-transfer agent according to claim 1, wherein the lipophilic, aromatic thiol comprising the general structure (IV) is selected from the group consisting of 4-dodecylthiophenol, O-dodecyl-4-hydroxythiophenol, S-dodecyl-1,4-benzenedithiol, N-dodecyl-4-aminothiophenol, 4-octadecylthiophenol, O-octadecyl-4-hydroxythiophenol, S-octadecyl-1,4-benzenedithiol, or N-octadecyl-4-aminothiophenol.

14. The prooxidative chain-transfer agent according to claim 1, wherein the infectious disease is a parasitic infection in a human or animal organism.

15. The prooxidative chain-transfer agent according to claim 14, wherein the parasitic infection is caused by a parasite selected from Acanthamoeba, Anisakis, Ascaris lumbricoides, Botfly, Balantidium coli, Bedbug, Cestoda (tapeworm), Chiggers, Cochliomyia hominivorax, Entamoeba histolytica, Fasciola hepatica, Giardia lamblia, Hookworm, Leishmania spp., Linguatula serrata, Liver fluke, Loa, Paragonimus - lung fluke, Pinworm, Plasmodium spp., Schistosoma spp., Strongyloides stercoralis, Mite, Tapeworm, Toxoplasma gondii, Trypanosoma spp., Whipworm, or Wuchereria bancrofti.

16. The prooxidative chain-transfer agent according to claim 1, wherein the malignant tumour disease is selected from the group consisting of breast cancer, leukemia, squamous cell cancer, small-cell lung cancer, non-small cell lung cancer, gastrointestinal cancer, pancreatic cancer, glioblastoma, cervical cancer, ovarian cancer, liver cancer, bladder cancer, hepatoma, colon cancer, colorectal cancer, endometrial carcinoma, salivary gland carcinoma, kidney cancer, liver cancer, prostate cancer, vulval cancer, thyroid cancer, hepatic carcinoma or head and neck cancer.

17. A prooxidative chain-transfer agent according to any one of claims 1 to 13 for use as medicament.
